Europäisches Patentamt

European Patent Office

Office européen des brevets

(11) Publication number: **0 285 389 B1**

## EUROPEAN PATENT SPECIFICATION

(45) Date of publication of patent specification: **23.09.92**   (51) Int. Cl.⁵: **A61K  7/08**

(21) Application number: **88302822.7**

(22) Date of filing: **30.03.88**

(54) Shampoo compositions.

(30) Priority: **10.04.87 US 36656**
**01.04.87 US 33443**

(43) Date of publication of application:
**05.10.88 Bulletin  88/40**

(45) Publication of the grant of the patent:
**23.09.92 Bulletin  92/39**

(84) Designated Contracting States:
**AT BE CH DE FR GB GR IT LI LU NL SE**

(56) References cited:
**EP-A- 0 181 773**
**EP-A- 0 190 010**
**EP-A- 0 240 350**
**DE-A- 3 637 683**
**GB-A- 2 178 443**

(73) Proprietor: **THE PROCTER & GAMBLE COM-
PANY
One Procter & Gamble Plaza
Cincinnati Ohio 45202(US)**

(72) Inventor: **Hutchinson, Neal Kevin
8510 Capricorn Drive
Montgomery Ohio 45249(US)**
Inventor: **Grote, Mark Bernard
3416 Ferncroft Drive
Cincinnati Ohio 45211(US)**
Inventor: **Dzialo, Kathleen Brown
1501 Governor Terrace
Cincinnati Ohio 45215(US)**

(74) Representative: **Brooks, Maxim Courtney et al
Procter & Gamble (NTC) Limited Whitley
Road Longbenton
Newcastle-upon-Tyne NE12 9TS(GB)**

## Description

The present invention is related to mild, good cleaning conditioning shampoos which contain a dispersed, non-volatile silicone phase and are stabilized through the use of certain long chain materials.

Human hair becomes soiled due to its contact with the surrounding atmosphere and, to a greater extent, from sebum secreted by the head. The build-up of the sebum causes the hair to have a dirty feel and an unattractive appearance. The soiling of the hair necessitates it being shampooed with frequent regularity.

Shampooing the hair cleans by removing excess soil and sebum. However, the shampooing process has disadvantages in that the hair is left in a wet, tangled and generally unmanageable state. A variety of approaches have been developed to alleviate the after-shampoo problems. These range from the inclusion of hair conditioning aids in shampoos to post-shampoo application of hair conditioners, i.e., hair rinses. Hair rinses typically work by depositing a polymeric film or other material onto the hair. However, such solutions to a very prevalent problem have not been fully satisfactory. For one thing, hair rinses must be applied in a separate step following the shampooing, left on the hair for a length of time, and rinsed with fresh water. This, of course, is time consuming and is not convenient.

While shampoos have been disclosed which contain conditioning aids, they have not been totally satisfactory for a variety of reasons. One problem relates to compatibility problems between good cleaning anionic surfactants and the fatty cationic agent which are good conditioning agents. This caused other surfactants such as nonionics, amphoterics and zwitterionics to be examined by workers in the field. Many of these efforts are reflected in patents issued in the conditioning shampoo area. See for example US-A-3,849,348, November 19, 1974 to Hewitt; US-A-3,990,991, November 9, 1961 to Gerstein; and US-A-3,822,312, July 2, 1974 to Sato.

The use of these other surfactants solved many of the compatibility problems but still did not provide complete answers in all areas. For instance cationic conditioners may not deliver the desired level of softness desired by users. Materials which can provide increased softness are silicones, both those which are soluble as well as insoluble in the shampoo matrix.

Silicones in shampoo compositions have been disclosed in a number of different publications. Such publications include US-A-2,826,551, March 11, 1958 to Geen; US-A-3,964,500, June 22, 1976 to Drakoff; US-A-4,364,837, December 21, 1982 to Pader; GB-A-0,849,433, September 28, 1960 to Woolston; and EP-A-0,240,350 in which a rigid silicone polymer is added to shampoo and other hair care compositions. While these patents disclose silicone containing compositions, they do not provide answers to all of the problems encountered in making a totally satisfactory product. One problem is that of keeping a dispersed, insoluble silicone material suspended and the total product stable. A second problem is related to making good cleaning, anionic surfactant based shampoos milder.

An approach which has shown to be beneficial in suspending silicone material involves the use of long chain acyl derivatives and other long chain derivatives. Such an approach is disclosed in published EP-A-0181773, published May 21, 1986 and in GB-A-2 178 443 in which xanthan gum is additionally added to suspend the silicone.

The present inventors having surprisingly found that by using a particular combination of alkyl ether sulfate and alkyl sulfate surfactants, shampoos which are milder than straight alkyl sulfate systems and yet lather well can be formed.

It is an object of the present invention therefore to provide a stable silicone containing conditioning shampoo.

It is a further object of the present invention to provide silicone shampoo compositions using anionic surfactants which lather well while being milder than straight alkyl sulfate surfactant systems.

These and other objects will become readily apparent from the detailed description which follows.

Unless otherwise indicated, all percentages and ratios herein are by weight.

The present invention relates to a shampoo composition comprising;

(a) from 10% to 40% by weight of a mixture of ethoxylated $C_8$-$C_{18}$ alkyl sulfate and $C_8$-$C_{18}$ alkyl sulfate wherein the ethoxylated alkyl sulfate is a sulfuric acid ester of the reaction product of one mole of $C_8$-$C_{18}$ alcohol and from 1 to 8 moles of ethylene oxide;

(b) from 0.01% to 10% by weight of a dispersed, insoluble, non-volatile silicone;

(c) from 0.5% to 5% by weight of a suspending agent selected from long-chain [$C_{16}$-$C_{22}$] acyl derivatives and mixtures thereof, said acyl derivative being present in the shampoo composition in the form of crystals; and

(d) water,

wherein the molar ratio of ethoxylated alkyl sulfate to alkyl sulfate is from 3:2 to 6:1.

The essential components of the present invention are given in the following paragraphs.

## Surfactant

An essential component of the present compositions is a combination of surfactants. The surfactant mixture is present at a level of from 10 to 40%, preferably from 10% to 30%, most preferably from 15% to 22%.

The surfactants used in the present compositions are ethoxylated alkyl sulfates and alkyl sulfates in a molar weight ratio of the former to the latter of from 3:2 to 6:1, preferably from 2.5:1 to 3.5:1.

The ethoxylated alkyl sulfates are salts, preferably the sodium, ammonium, potassium or triethanolomine salts, of sulfuric acid esters of the reaction product of one mole of higher alcohols ($C_8$-$C_{18}$ carbon atoms, linear as well as branched) and one to eight moles of ethylene oxide.

The alkyl sulfates can be exemplified by those prepared by sulfating the higher fatty alcohols ($C_8$-$C_{18}$ carbon atoms) and forming the sodium, potassium, ammonium, or triethanolomine salt.

Specific preferred alkyl sulfates and ethoxylated alkyl sulfates are ammonium lauryl sulfate and ammonium laureth-3-sulfate.

## Non-Volatile Silicone

Silicone fluids are a suitable non-volatile silicone that may be used in the present compositions.

The non-volatile silicone fluid may be either a polyalkyl siloxane, a polyaryl siloxane, a polyalkylaryl siloxane or a polyether siloxane copolymer and is present at a level of from 0.01% to 10% preferably from 0.5% to 3%. Mixtures of these fluids may also be used and are preferred in certain executions. The dispersed silicone particles should also be insoluble in the shampoo matrix. This is the meaning of "insoluble" as used hereinbefore and hereinafter.

The essentially non-volatile polyalkyl siloxane fluids that may be used include, for example, polydimethyl siloxanes with viscosities ranging from 5 to 600,000 $mm^2 \cdot s^{-1}$ (centistokes) at 25°C. These siloxanes are available, for example, from the General Electric Company as the Viscasil (RTM) series and from Dow Corning as the Dow Corning 200 series. The viscosity can be measured by means of a glass capillary viscometer as set forth in Dow Corning Corporate Test Method CTM0004, July 20, 1970. Preferably the viscosity ranges from 350 $mm^2 \cdot s^{-1}$ (centistokes) to 100,000 $mm^2 \cdot s^{-1}$ (centistokes).

The essentially non-volatile polyalkylaryl siloxane fluids that may be used include, for example, polymethylphenylsiloxanes having viscosities of 15 to 30,000 $mm^2 \cdot s^{-1}$ (centistokes) at 25°C. These siloxanes are available, for example, from the General Electric Company as SF 1075 methyl phenyl fluid or from Dow Corning as 556 Cosmetic Grade Fluid.

The essentially non-volatile polyether siloxane copolymer that may be used is, for example, a polypropylene oxide modified dimethylpolysiloxane (e.g., Dow Corning DC-1248) although ethylene oxide or mixtures of ethylene oxide and propylene oxide may also be used.

References disclosing suitable silicone fluids include the previously mentioned US-A-2,826,551 to Geen; US-A-3,964,500, June 22, 1976 to Drakoff; US-A-4,364,837 to Pader and GB-A-0,849,433 to Woolston. Silicon Compounds distributed by Petrarch Systems, Inc., 1984 also provides a very good listing of suitable silicone materials.

Another silicone material found especially useful in the present compositions to provide good dry combing is a silicone gum. Silicone gums described by Petrarch and others including US-A-4,152,416, May 1, 1979 to Spitzer, et al. and Noll, Walter, Chemistry and Technology of Silicones, New York: Academic Press 1968. Also describing silicone gums are General Electric Silicone Rubber Product Data Sheets SE 30, SE 33, SE 54 and SE 76. "Silicone gum" materials denote high molecular weight polydiorganosiloxanes having a mass molecular weight of from 200,000 to 1,000,000. Specific examples include polydimethylsiloxane, (polydimethylsiloxane) (methylvinylsiloxane) copolymer, poly(dimethylsiloxane) (diphenyl) (methylvinylsiloxane) copolymer and mixtures thereof.

## Long Chain Derivative Suspending Agent

The suspending agent useful in the present compositions can be any of several long chain acyl derivative materials or mixtures of such materials. Included are ethylene glycol esters of fatty acids having from 16 to 22 carbon atoms. Preferred are the ethylene glycol stearates, both mono and distearate, but particularly the distearate containing less than about 7% of the mono stearate. Other suspending agents found useful are alkanol amides of fatty acids, having from 16 to 22 carbon atoms, preferably 16 to 18 carbon atoms. Preferred alkanol amides are stearic monoethanolamide, stearic diethanolamide, stearic monoisopropanolamide and stearic monoethanolamide stearate. Other long chain acyl derivatives include long chain esters of long chain fatty acids (e.g., stearyl stearate, cetyl palmitate, etc); glyceryl esters (e.g., glyceryl distearate) and long chain esters of long chain alkanol amides (e.g., stearamide DEA distearate).

The suspending agent is present at a level of from 0.50% to 5.0%, preferably from 0.5% to 3.0%. The suspending agent serves to assist in suspending the silicone material and may give pearlescence to the product. Mixtures of suspending agents are also suitable for use in the compositions of this invention.

Water

Water is the last essential component of the present invention and forms the remainder of the composition. It is generally present at a level of from 20% to 95%, preferably from 60% to 85%.

Optional Components

The shampoos herein can contain a variety of non-essential optional components suitable for rendering such compositions more acceptable. Such conventional optional ingredients are well known to those skilled in the art, e.g., preservatives such as benzyl alcohol, methyl paraben, propyl paraben and imidazolidinyl urea; cationic surfactants such as cetyl trimethyl ammonium chloride, lauryl trimethyl ammonium chloride, tricetyl methyl ammonium chloride, stearyldimethyl benzyl ammonium chloride, and di(partially hydrogenated tallow) dimethylammonium chloride; thickeners and viscosity modifiers such as a diethanolamide of a long chain fatty acid (e.g., PEG 3 lauramide), block polymers of ethylene oxide and propylene oxide such as Pluronic (RTM) F88 offered by BASF Wyandotte, ammonium xylene sulfonate, sodium chloride, sodium sulfate, polyvinyl alcohol, and ethyl alcohol; pH adjusting agents such as citric acid, succinic acid, phosphoric acid, sodium hydroxide, sodium carbonate, etc.; perfumes; dyes; and, sequestering agents such as disodium ethylenediamine tetraacetate. Such agents generally are used individually at a level of from 0.01% to 10%, preferably from 0.5% to 5.0% by weight of the composition.

Another optional component is an additional surfactant used in combination with the alkyl sulfate/ethoxylated alkyl sulfate mixture. Included among optional surfactants are the sodium alkyl glyceryl ether sulfonates; the reaction product of fatty acids having from 10 to 22 carbon atoms esterified with isethionic acid and neutralized with sodium hydroxide; water soluble salts of condensation products of fatty acids with sarcosine; among several others. The optional surfactants, collectively, can be used in any amount (molar amount) up to the molar amount of the alkyl sulfate ethoxylated alkyl sulfate blend.

Still another optional component of the present invention is an antidandruff agent. Suitable agents include sulfur, selenium sulfide, salicylic acid, zinc pyridinethione, other 1-hydroxy pyridones, such as those disclosed in US-A-4,185,106, Jan. 22, 1980 to Dittmar et al and azole antimycotics, disclosed in GB-A-1,502,144, Feb. 22, 1978, among many others. The antidandruff agent is preferably present at a level of from 0.2% to 4%.

Zinc pyridinethione is the preferred agent, particularly where its salt crystals are predominantly flat platelets which have a mean sphericity less than 0.65 preferably between 0.20 and 0.65, and a median particle diameter of at least $2\mu$m, expressed as the diameter of a sphere of equivalent volume. It is preferred that the median particle diameter not be greater than about $15\mu$m, expressed on the same basis.

The diameter of a sphere of equivalent volume, $d_\nu$, for a particle can be determined by a variety of sedimentation techniques which are based on Stokes' Law for the settling velocity of a particle in a fluid. Such techniques are described in Stockham, J.D. and Fochtman, E.G., "Particle Size Analysis", Ann Arbor Science, 1978.

The sphericity of a particle is also described by Stockham and Fochtman at page 113 as

$$\psi = \left( \frac{\bar{d}_\nu}{\bar{d}_s} \right)^2$$

where $d_\nu$ is the diameter of a sphere of equivalent volume, supra, and $d_s$ is the diameter of a sphere of equivalent area. A technique for determining $d_s$ is the BET technique described by Stockham and Fochtman at page 122.

Since the sphericity of interest herein is the mean sphericity, the mean diameters are employed.

Other optional components include other suspending agents such as xanthan gum at a level of from 0.2% to 5% and shorter chain amides such as mono and diethanolamides of fatty acids having from 8 to 14 carbon atoms commonly used in shampoos. Preferred are coconut monoethanalamide, lauric diethanolamide and mixtures thereof at a level of from 1% to 5%.

The pH of the present composition is not critical and may be in the range of from 2 to 10.

The shampoos of the present invention can be made by mixing a portion of the surfactant blend, the suspending agent and the silicone together and heating to about 72°C. The mixture is mixed thoroughly for about 10 minutes at the 72°C temperature before being pumped through a high shear mill and then through a heat exchanger to cool it to below about 35°C. The remainder of the composition is added at this time and the total composition mixed.

The high shear mill is used to achieve adequate dispersion of the silicone fluid. This is achieved by having the average particle size of the silicones preferably be 10$\mu$m or less.

In the cooling step, the acyl derivative is preferably crystallized into particles having an average particle size of 10$\mu$m or less.

The present compositions are used in a conventional manner for cleaning hair. From about 0.1g to about 10g of a composition is applied to hair that has been wetted, generally with water, worked through the hair and then rinsed out.

The following Examples further describe and demonstrate the preferred embodiments within the scope of the present invention.

EXAMPLES I-VII

The following are compositions representative of the present invention.

| Component | | Weight % | | | | | |
|---|---|---|---|---|---|---|---|
| | I | II | III | IV | V | VI | VII |
| Water | qs to 100% ——————————————————————> | | | | | | |
| Ammonium Laureth-3 Sulfate (28%) | 50.5 | 50.5 | 38.6 | 50.5 | 50.5 | 25.2 | 41.6 |
| Ammonium Lauryl Sulfate (25%) | 12.6 | 12.6 | 9.6 | 25.2 | 6.3 | 12.6 | 10.4 |
| Cocomide MEA | 3.0 | 3.0 | 3.0 | 4.0 | 3.0 | 2.0 | 2.0 |
| Glycol Distearate | 3.0 | 3.0 | 3.0 | 2.0 | 4.0 | 5.0 | 3.0 |
| Dimethicone[1] | 1.0 | 1.0 | 1.0 | 0.5 | 1.5 | 2.5 | 1.0 |
| Sodium Cocoyl Isethionate (85%) | – | – | 4.7 | – | – | 9.4 | – |
| Zinc Pyrithione (25%) | – | 4.0 | 4.0 | – | – | – | 4.0 |

| | | | | | | | |
|---|---|---|---|---|---|---|---|
| Sodium Cocogylceryl Ether Sulfonate (50%) | – | – | – | – | – | – | 6.0 |
| Color, preservative, pH control, viscosity control, others | qs | qs | qs | qs | qs | qs | qs |
| pH, ± 0.2 | 5.5 | 6.0 | 6.5 | 7.5 | 7.0 | 4.5 | 6.0 |
| AES/AS Ratio | 3:1 | 3:1 | 3:1 | 3:2 | 6:1 | 3:2 | 3:1 |

1  Dimethicone having viscosity of $10^6$ *mPa.s* (cps)

The above compositions are both mild and lather well.

In the above compositions other surfactants such as sodium myreth-3 sulfate or TEA laureth-2 sulfate may be used in place of ammonium laureth-3 sulfate. Similarly, sodium myristyl sulfate or TEA lauryl sulfate may be used in place of ammonium lauryl sulfate. Additionally, other silicones may be used in place of the material shown such as lower or higher viscosities.

**Claims**

**1.** A shampoo composition comprising;

(a) from 10% to 40% by weight of a mixture of ethoxylated $C_8$-$C_{18}$ alkyl sulfate and $C_8$-$C_{18}$ alkyl sulfate wherein the ethoxylated alkyl sulfate is a sulfuric acid ester of the reaction product of one mole of $C_8$-$C_{18}$ alcohol and from 1 to 8 moles of ethylene oxide;

(b) from 0.01% to 10% by weight of a dispersed, insoluble, non-volatile silicone;

(c) from 0.5% to 5% by weight of a suspending agent selected from long-chain [$C_{16}$-$C_{22}$] acyl derivative or mixtures thereof, said acyl derivative being present in the shampoo composition in the form of crystals; and

(d) water,

wherein the molar ratio of ethoxylated alkyl sulfate to alkyl sulfate is from 3:2 to 6:1.

**2.** A shampoo composition according to Claim 1 wherein the long chain acyl derivative is selected from ethylene glycol long chain esters, alkanol amides of long chain fatty acids, long chain esters of long chain fatty acids, glyceryl long chain esters, long chain esters of long chain alkanolamides, or mixtures thereof.

**3.** A shampoo composition according to Claim 1 or 2 wherein the non-volatile silicone is selected from polydimethylsiloxanes having viscosities of from 5 to 100,000 $mm^2.s^{-1}$(centistokes)at 25° C, polypropylene oxide modified dimethylsiloxanes, silicone gums and mixtures thereof.

**4.** A shampoo composition according to any of Claims 1 to 3 which in addition contains a mono- or diethanolamide of a fatty acid having from 8 to 14 carbon atoms.

**5.** A shampoo composition according to any of Claims 1 to 4 which in addition contains an isethionate surfactant salt.

**6.** A shampoo composition according to any of Claims 1 to 5 wherein the long chain acyl derivative is an ethylene glycol long chain ester.

**Patentansprüche**

**1.** Shampoozusammensetzung, umfassend:

(a) von 10 Gew.-% bis 40 Gew.-% eines Gemisches aus ethoxyliertem $C_8$-$C_{18}$-Alkylsulfat und $C_8$-$C_{18}$-Alkylsulfat, worin das ethoxylierte Alkylsulfat ein Schwefelsäureester des Reaktionsproduktes von einem Mol $C_8$-$C_{18}$-Alkohol mit 1 bis 8 Molen Ethylenoxid ist;

6

(b) von 0,01 Gew.-% bis 10 Gew.-% eines dispergierten, unlöslichen, nicht-flüchtigen Silikons;

(c) von 0,5 Gew.-% bis 5 Gew.-% eines Suspendiermittels, ausgewählt unter langkettigen [$C_{16}$-$C_{22}$]-Acylderivaten oder Gemischen hievon, wobei das Acylderivat in der Shampoozusammensetzung in Form von Kristallen vorliegt; und

(d) Wasser,

wobei das Molverhältnis von ethoxyliertem Alkylsulfat zu Alkylsulfat von 3:2 bis 6:1 beträgt.

2. Shampoozusammensetzung nach Anspruch 1, worin das langkettige Acylderivat unter Ethylenglykol-langkettigen Estern, Alkanolamiden von langkettigen Fettsäuren, langkettigen Estern von langkettigen Fettsäuren, Glyceryl-langkettigen Estern, langkettigen Estern von langkettigen Alkanolamiden oder Gemischen hievon ausgewählt ist.

3. Shampoozusammensetzung nach Anspruch 1 oder 2, worin das nicht-flüchtige Silikon unter Polydimethylsiloxanen mit Viskositäten von 5 bis 100.000 $mm^2.s^{-1}$ (Centistoke) bei 25°C, Polypropylenoxid-modifizierten Dimethylsiloxanen, Silikongummis und Gemischen hievon ausgewählt ist.

4. Shampoozusammensetzung nach einem der Ansprüche 1 bis 3, die zusätzlich ein Mono- oder Diethanolamid einer Fettsäure mit 8 bis 14 Kohlenstoffatomen enthält.

5. Shampoozusammensetzung nach eine der Ansprüche 1 bis 4, die zusätzlich ein grenzflächenaktives Isethionatsalz enthält.

6. Shampoozusammensetzung nach einem der Ansprüche 1 bis 5, worin das langkettige Acylderivat ein Ethylenglykol-langkettiger Ester ist.

**Revendications**

1. Composition de shampooing comprenant :

a) de 10 à 40% en poids d'un mélange d'un sulfate d'alkyle en $C_8$ à $C_{18}$ éthoxylé et d'un sulfate d'alkyle en $C_8$ à $C_{18}$, dans lequel le sulfate d'alkyle éthoxylé est un ester sulfurique du produit de la réaction d'une mole d'un alcool en $C_8$ à $C_{18}$ avec 1 à 8 moles d'oxyde d'éthylène ;

b) de 0,01% à 10% en poids d'une silicone non volatile insoluble et dispersée ;

c) de 0,5 à 5% en poids d'un agent de mise en suspension choisi parmi les dérivés acyle à longue chaîne ($C_{16}$ à $C_{22}$) ou leurs mélanges, ce dérivé étant présent dans la composition de shampooing sous forme de cristaux ;

d) de l'eau,

dans laquelle le rapport molaire du sulfate d'alkyle éthoxylé et du sulfate d'alkyle est de 3:2 à 6:1.

2. Composition de shampooing selon la revendication 1, dans laquelle le dérivé acyle à longue chaîne est pris dans le groupe comprenant des esters à longue chaîne d'éthylèneglycol, des alcanol-amides d'acides gras à longue chaîne, des esters à longue chaîne d'acides gras à longue chaîne, des esters à longue chaîne glycériques, des esters à longue chaîne d'alcanolamides à longue chaîne ou des mélanges de ceux-ci.

3. Composition de shampooing selon la revendication 1 ou 2, dans laquelle la silicone non volatile est prise dans le groupe comprenant des polydiméthylsiloxanes ayant des viscosités de 5 à 100 000 $mm^2/s$ (centistokes) à 25°C, des diméthylsiloxanes modifiés par l'oxyde de polypropylène, des gommes de silicone et des mélanges de ceux-ci.

4. Composition de shampooing selon l'une quelconque des revendications 1 à 3, contenant de plus un mono- ou di-éthanolamide d'un acide gras ayant de 8 à 14 atomes de carbone.

5. Composition de shampooing selon l'une quelconque des revendications 1 à 4 contenant en plus un sel tensioactif d'isethionate.

6. Composition de shampooing selon l'une quelconque des revendications 1 à 5 dans laquelle le dérivé acyle à longue chaîne est un ester à longue chaîne d'éthylèneglycol.